# EUROPEAN PATENT SPECIFICATION

(11) **EP 1 292 249 B1**
(45) Date of publication and mention of the grant of the patent: **23.11.2005**
(21) Application number: 01932713.9
(22) Date of filing: 30.04.2001
(51) Int. Cl.: A61F 2/36

(54) **TISSUE ENGINEERED STENTS**
AUS GEWEBE AUFGEBAUTER STENT
TUTEURS MIS AU POINT PAR GENIE TISSULAIRE

(30) Priority: 28.04.2000 US 200427 P
(43) Date of publication of application: 19.03.2003
(73) Proprietor: CHILDREN'S MEDICAL CENTER CORPORATION, Boston Massachusetts 02115 (US)
(72) Inventor: ATALA, Anthony, Weston, MA 02193 (US)
(74) Representative: Ouzman, Beverley Nicola Claire
(86) International application number: PCT/US2001/013679
(87) International publication number: WO 2001/087192

(56) References cited:
- WO-A-99/62430
- US-A- 4 553 272
- US-A- 5 749 922
- US-A- 5 858 721
- US-A- 6 153 292
- US-B1- 6 176 874
- US-B1- 6 245 537

## Description

### FIELD OF THE INVENTION

The present invention is generally directed to tissue engineering. More particularly, the present invention provides a tissue-engineered stent useful in the treatment of strictures.

### BACKGROUND OF THE INVENTION

Cylindrical organs and structures such as blood vessels the esophagus, intestines, endocrine gland ducts and the urethra are all subject to strictures i.e., a narrowing or occlusion of the lumen. Strictures can be caused by a variety of traumatic or organic disorders and symptoms can range from mild irritation and discomfort to paralysis and death.

Urethral strictures can be congenital or acquired. Acquired urethral stricture is common in men but rare in women. Most acquired strictures are due to infection or trauma. Apart from infections caused by venereal diseases, infection from long term use of urethral catheters and the use of large caliber instruments inserted for medical uses into the urethra causes trauma to the urethra. External trauma, e.g., pelvic bone fractures or saddle injuries, can also cause urethral strictures. The narrowing of the lumen restricts the urine flow. In chronic cases the bladder muscle becomes hypertrophic, and later an increase in the residual urine may develop in the bladder. Prolonged obstruction may cause incompetence of the outflow control mechanism resulting in incontinence or high pressures in the bladder resulting in kidney damage and renal failure. Residual urine may be a predisposing factor for urinary infections which include prostatic infections, urethral abscess and also bladder stones.

A full description of the pathology of the male urethra is provided in Campbell's Urology, 5th Edition, 1986, Vol. 1, pages 520-523 and 1217-1234, and Vol. 3, pages 2860-2886 (published by W. B. Saunders Co., Philadelphia, Pa. U.S.A.).

Treatment of strictures is site specific and varies with the nature and extent of the occlusion. Urethral strictures can be managed with palliative treatments such as dilatations of the urethra, which are not curative, because dilatation fractures the scar tissue and temporarily enlarges the lumen. As healing occurs, the scar tissue reforms.

Visually controlled internal urethrotomy is also used in the treatment of urethral strictures. However, in most cases the stricture reoccurs and the procedure has to be repeated.

Plastic surgical repair of the stricture is a meticulous and complicated procedure. However, this procedure has a high recurrence of urethral strictures, and because of the lack of enough experienced surgeons for reconstructive surgery, the majority of cases are managed by non-curative methods.

Previous studies have shown that nearly one-half to two-thirds of urethral and ureteral strictures recur within the first year after treatment.¹⁰⁻¹⁵ Several materials have been proposed for permanent replacement in the treatment of stricture disease in the genitourinary tract. Permanent metallic stents and temporary metallic and polyurethane stents were introduced in the last two decades.¹⁶⁻¹⁸ However, due to disappointing long-term results, these devices are being utilized only in limited situations. ^{14,19,20}

In typical urethral stenting procedures, the stent is transurethrally inserted and positioned within the desired portion of the urethra. In order for a stent to be used most advantageously, it is desirable for the stent to have a delivery diameter that is substantially smaller than the diameter of the body lumen. Upon deployment, the stent should assume a deployed diameter that is essentially equal to the diameter of the body lumen.

The use of stents in the urethra creates special problems. Minerals suspended in the urine can cause encrustation on the stent internal walls that may eventually block the flow of urine through the stent. Epithelialization, where the cells of the urethra grow over the stent, can complicate or even prevent the removal of the stent. Undesirable movement, also known as migration, of the stent from its desired location may prevent the stent from properly supporting the desired portion of the urethra. In some cases, a migrating stent can block or injure the urethra itself. A migrating stent may also cause incontinence, such as when a stent migrates to a position in the urethra between the distal sphincter.

Examples of intraurethral and other intraluminal stents can be found in U.S. Pat. Nos. 4,740,207 (Kreamer); 4,877,030 (Beck et al.); 5,059,211 (Stack et al.); 5,078,726 (Kreamer); 5,192,307 (Wall); 5,306,294 (Winston et al.); 5,354,309 (Schnepp-Pesch et al.); and 5,383,926 (Lock et al.).

In 1988, Milroy et al reported the successful use of a self expandable metallic urethral Stent.¹⁶ Shortly thereafter, these stents were noted to induce local pain and discomfort, hypertrophic proliferation of the epithelium, and obstruction of the lumen in many patients. These symptoms required repeated endoscopic resections or even surgical removal of the stents, necessitating further surgical reconstruction. Bioabsorbable urethral stents have also been used for recurrent urethral strictures, but the results were disappointing.²¹ Epithelial hyperplasia and obstructing fibrosis within the stents led to failure.²² Since current treatment modalities for urethral and ureteral strictures are suboptimal and recurrence rates are high, novel treatment modalities need to be explored.

### SUMMARY OF THE INVENTION

The use of natural stents made of autologous tissue would be advantageous for treatment of urethral and ureteral strictures due to their biocompatibility and their ability to withstand high compression forces, which would allow them to be functional in the urinary tract. It is therefore a primary object of the present invention to provide an effective device for use in treatment of stricture diseases. The stent according to the present invention can be useful in stricture diseases involving urethra, ureters, blood vessels, biliary ducts, intestines, airways of the lungs, and fallopian tubes. The attending physician will decide to what extent the new device can be employed in treatment of stenotic conditions of a duct in a human body.

It is a further object of this invention to provide a urethral or other intraluminal stents which effectively treats stricture and prevents it from recurring.

These and other objects of the present invention are achieved, according to the invention by a tissue-engineered stent comprising a substantially cylindrical construct having a first end and a second end; a walled surface disposed between the first end and the second end; the walled surface comprising a biodegradable polymer scaffold seeded with disassociated chondrocytes. The seeded scaffold is preferably cultured *in vitro* prior to implantation in a host. Preferably, the culturing is for a time period sufficient for cartilaginous tissue to form.

The chondrocytes can be autologous, allogenic or xenogenic. Autologous chondrocytes are preferred.

The term "biodegradable", as used herein refers to materials which are enzymatically or chemically degraded *in vivo* into simpler chemical species. Either natural or synthetic polymers can be used to form the matrix, although synthetic biodegradable polymers are preferred for reproducibility and controlled release kinetics. Synthetic polymers that can be used include polymers such as poly(lactide) (PLA), poly(glycolic acid) (PGA), poly(lactide-co-glycolide) (PLGA), poly(caprolactone), polycarbonates, polyamides, polyanhydrides, polyamino acids, polyortho esters, polyacetals, polycyanoacrylates and degradable polyurethanes, and non-erodible polymers such as polyacrylates, ethylene-vinyl acetate polymers and other acyl substituted cellulose acetates and derivatives thereof. PGA and PLGA are preferred.

Preferred biodegradable polymers comprise a polymer selected from the group consisting of polyesters of hydroxycarboxylic acids, polyanhydrides of dicarboxylic acids, and copolymers of hydroxy carboxylic acids and dicarboxylic acids. In other embodiments the material is a synthetic polymer derived from at least one of the following monomers: glycolide, lactide, p-dioxanone, caprolactone, trimethylene carbonate, butyrolactone. In preferred embodiments, the material is selected from the group consisting of polymers or copolymers of glycolic acid, lactic acid, and sebacic acid. Polyglycolic acid polymers are most preferred. A polyhydroxyalkanoate (PHA) polymer may also be used.

In further embodiment, the present invention provides a method for producing a tissue engineered stent. The method provides the steps of:
(a) providing a substrate shaped to form a substantially cylindrical construct, the substrate comprising biodegradable polymer;
(b) contacting said substrate with dissociated chondrocytes capable of adhering thereto and forming cartilage, thereby forming a cell-seeded construct;
(c) maintaining said cell-seeded construct for a growth period in a fluid media suitable for growth of said chondrocytes to form a tissue-engineered stent.

In another embodiment, stresses are imparted to the cell-seeded construct during the growth phase to stimulate physiological conditions to be encountered by the stent once implanted. Preferably, the stresses are cyclic increases in pressure.

Other aspects of the invention are disclosed *infra.*

### BRIEF DESCRIPTION OF THE FIGURES

For a more complete understanding of the invention, reference should be made to the figures, in which:

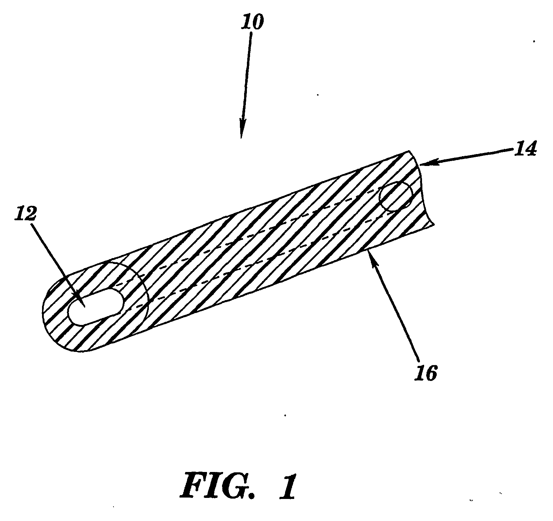

Figure 1 shows a substantially cylindrical construct (10) having a first end (12) and a second end (14); a walled surface (16) disposed between its first end and its second end.
Figure 2A is a scanning electron microscopy of Polymer matrix prior to seeding (x200) and Figure 2B is Polymer matrix seeded with 60x10⁶ chondrocytes/cc 72 hrs after seeding (x200).
Figure 3A depicts tubularized polymer matrix prior to seeding and Figure 3B depicts a Cartilaginous urethral stent grown in a bioreactor at 10 weeks.

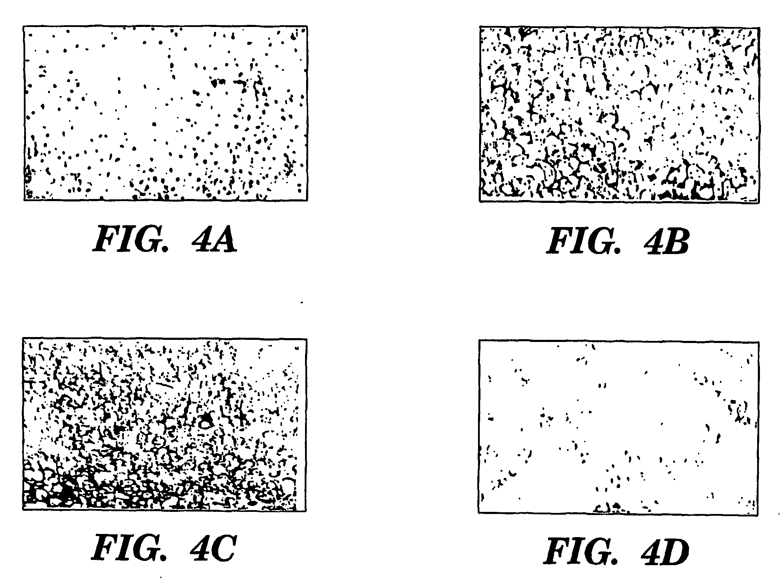

Figures 4A-4D show histological sections of engineered cartilage stents. PGA meshes seeded with bovine chondrocytes and grown in a bioreactor in vitro at 4 weeks (Figure 4A) and 10 weeks (Figure 4B), implanted in the subcutaneous space of nude mice at 4 weeks (Figure 4C) and 10 weeks (Figure 4D). Sections were stained with hematoxylin & eosin (x250).
Figures 5A-5F show histological sections of engineered cartilage stents. Masson's trichrome staining for collagen is shown at 10 weeks in vitro (x250) in Figure 5A and at 10 weeks in vivo (x250) in Figure 5B. Safranin-O staining for GAG is shown at 10 weeks in vitro (x250) in Figure 5C and at 10 weeks in vivo (x250) in Figure 5D. Alcian blue staining for chondroitin sulfate is shown at 10 weeks in vitro (x250) in Figure 5E and at 10 weeks in vivo (x250) in Figure 5F.
Figure 6 is a collagen content measurement using the Sircol Collagen Assay (Biocolor; Belfast, N. Ireland) *in vitro* and *in vivo.* Stents grown in vitro had an increase in collagen content of 94% between the 4-week and 10-week time points (3.6%;s.d.±0.22%. vs 7%;s.d.±0.41% respectively). Stents grown in vivo had over a three-fold increase in collagen content between the 4-week and 10-week time points (4%;s.d.±0.33% vs 12.4%;s.d.±0.91% respectively).
Figure 7 depicts compression forces required to reduce the diameter of the cartilage stent grown *in vitro* by 20% at 4 weeks (a) and 10 weeks (b).
Figure 8 depicts compression forces required to reduce the diameter of the cartilage stent grown *in vivo* by 20% at 4 weeks (a) and 10 weeks (b).

### DETAILED DESCRIPTION OF THE INVENTION

Trauma, operations or instrumentation of the urethra or ureter, may lead to stricture disease in the adult and pediatric populations. The present invention provides a tissue-engineered stent comprising a substantially cylindrical construct (10) having a first end (12) and a second end (14); a walled surface (16) disposed between its first end and its second end; the walled surface comprising a biodegradable polymer scaffold seeded with disassociated chondrocytes. The seeded scaffold is preferably cultured *in vitro* prior to implantation in a host.

Preferred biodegradable polymers include polyglycolic and acid polymers (PGA), polylactic acid polymers (PLA), polysebacic acid polymers (PSA), poly(lactic-co-glycolic) acid copolymers (PLGA), poly(lactic-co-sebacic) acid copolymers (PLSA), poly(glycolic-co-sebacid) acid copolymers (PGSA), and polyhydroxyalkanoate (PHA). PHAs and their production are described in, for example, PCT Publication Nos. WO99/14313, WO99/32536 and WO00/56376. Combinations of biodegradable polymers, e.g., PGA and PLGA, can be used.

Other biodegradable polymers useful in the present invention include polymers or copolymers of caprolactones, carbonates, amides, amino acids, orthoesters, acetals, cyanoacrylates and degradable urethanes, as well as copolymers of these with straight chain or branched, substituted or unsubstituted, alkanyl, haloalkyl, thioalkyl, aminoalkyl, alkenyl, or aromatic hydroxy- or di-carboxylic acids. In addition, the biologically important amino acids with reactive side chain groups, such as lysine, arginine, aspartic acid, glutamic acid, serine, threonine, tyrosine and cysteine, or their enantiomers, may be included in copolymers with any of the aforementioned materials.

Examples of synthetic biodegradable polymers are described in U.S. Patent Nos.: 5,431,679; 5,403,347; 5,314,989; and 5,502,159.

Surface properties of any medical device are extremely important, since it is this surface that interacts with the host. Since this invention employs cell seeded scaffolds for tissue engineering, it is not only necessary for the scaffold to be biocompatible and biodegradable, it is also essential that the surface is conducive to cell attachment and subsequent tissue growth. It is therefore desirable to be able to adjust surface properties to suit the intended application, without altering other properties of the scaffold such as its mechanical strength or thermal properties. Useful surface modifications could include, for example, changes in chemical group functionality, surface charge, hydrophobicity, hydrophilicity, and wettability. For example, it would be desirable to improve or maximize cellular attachment or allow for the attachment of the desired cell type or types. This can be accomplished, for example, by attaching or coating the surface with a bioactive compound or peptide which promotes cellular attachment. The coating or bioactive compound may be attached to the surface either covalently or non-covalently. Such skills are well known in the art.

The biodegradable polymer scaffold is configured to form a substantially cylindrical configuration.

Sterilization is performed prior to seeding the scaffold with chondrocytes. Heat sterilization is often impractical since the heat treatment could deform the device, especially if the materials have a melting temperature below that required for the heat sterilization treatment. This problem can be overcome using cold ethylene oxide gas as a sterilizing agent.

The scaffold is seeded with the chondrocytes prior to implantation. The chondrocytes may be harvested from a healthy section of the individuals tissue, e.g., articular cartilage or epiphysial growth-plate, preferably the ear, expanded *in vitro* using cell culture techniques and then seeded onto the scaffold. Alternatively, the chondrocytes may be obtained from other donor's tissues or from existing cell lines. Mesenchymal cells obtained from bone marrow can also be differentiated into chondrocytes under appropriate culture conditions as described by, e.g., Butnariu-Ephrat et al., Clinical Orthopaedics and Related Research, 330:234-243, 1996. Other sources from which chondrocytes can be derived include dermal cells and pluripotent stem cells.

The chondrocytes may be seeded onto the scaffold of the invention by any standard method.

Suitable growth conditions and media for cells in culture are well known in the art. Cell culture media typically comprise essential nutrients, but also optionally include additional elements (e.g., growth factors, salts and minerals) which may be customized for the growth and differentiation of particular cell types. In the preferred embodiment, cell-polymer constructs were suspended in HAMM's F12 medium (Gibco; New York, NY) containing 10% fetal bovine serum with L-glutamine (292 µg/ml), penicillin (100 µg/ml) and ascorbic acid (50 µg/ml). Other media may also be used. For example, "standard cell growth media" include Dulbecco's Modified Eagles Medium, low glucose (DMEM), with 110 mg/L pyruvate and glutamine, supplemented with 10-20% Fetal Bovine Serum (FBS) or 10-20% calf serum (CS) and 100U/ml penicillin. Other standard media include Basal Medium Eagle, Minimal Essential Media, McCoy's 5A Medium, and the like, preferably supplemented as above (commercially available from, e.g., JRH Biosciences, Lenexa, KS; GIBCO, BRL, Grand Island, NY; Sigma Chemical Co., St. Louis, MO).

The cell seeded construct may be placed in a bioreactor to form a tissue-engineered stent or implanted directly. A bioreactor containing a growth chamber having a substrate on which the chondorcytes are attached, and means for applying relative movement between a liquid culture medium and the substrate to provide the shear flow stress can be used. See, e.g., U.S. Pat. No. 5,928,945. The bioreactor works by applying sheer flow stress to the cells housed in a growth chamber thereby allowing a continuous flow of liquid growth medium from the reservoir of the medium through the growth chamber, and back to the reservoir, in response to force applied by a pump. If desired, the bioreactor can subject the device to changes in pressure.

The tissue-engineered stent is surgically implanted using standard surgical techniques.

It is to be understood that while the invention has been described in conjunction with the preferred specific embodiments thereof that the foregoing description as well as the examples that follow are intended to illustrate and not limit the scope of the invention.

### EXAMPLES

### Polymer Stent Construction

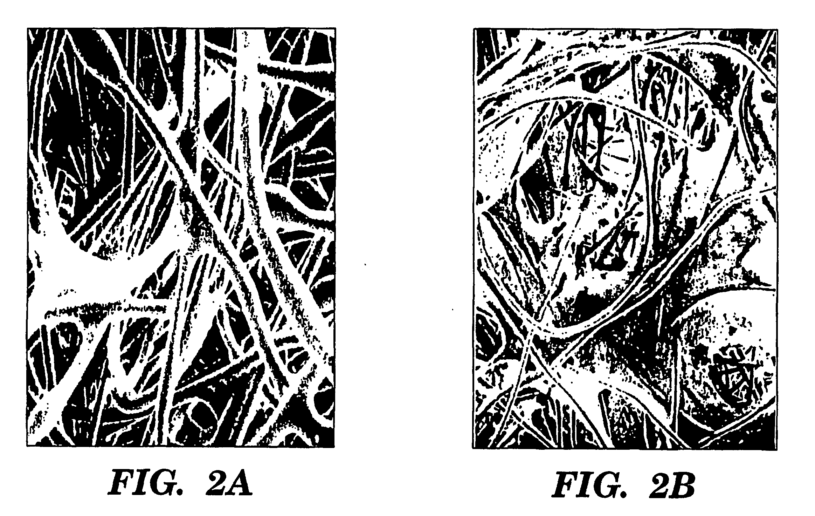

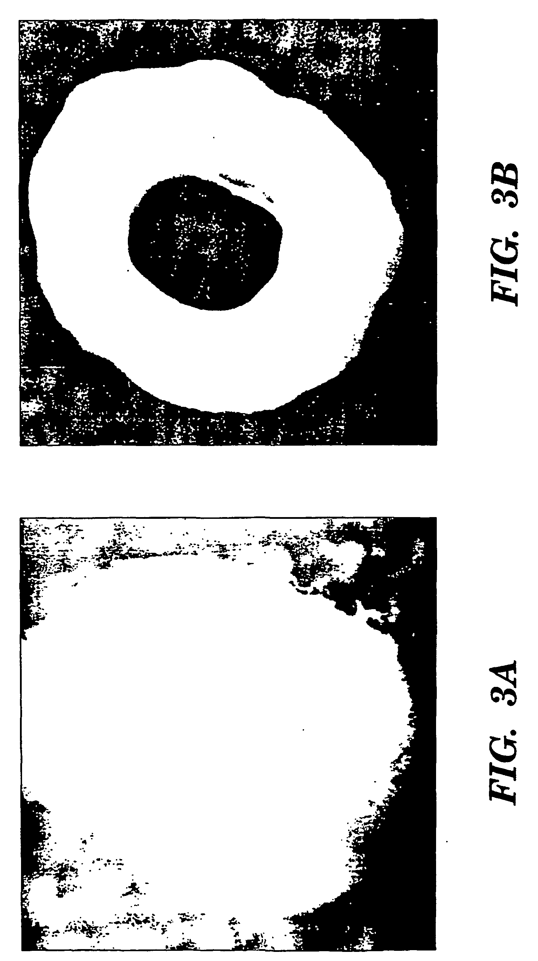

Non-woven meshes (2 mm thick) of PGA fibers were used to form the cylindrical constructs (Figure 2A). Sixteen french silicone foley catheters (Bard; Covington, GA) were wrapped once (360°) with the PGA sheets. The constructs were sprayed with a 50:50 poly-Lactic-co-glycolic acid (PLGA) solution (Sigma; St. Louis, MO) in chloroform to glue the edges of the PGA mesh and to maintain the circumferencial shape of the catheter. The constructs were 10mm long, with an inner diameter of 5mm, and an outer diameter of 9mm (Figure 3A).

### Cell harvesting and seeding

Full-thickness articular cartilage was harvested under sterile conditions from shoulders of 2-3 week-old bovine calves obtained from a local abattoir within 8h of slaughter. Chondrocytes were isolated by digestion with collagenase type II (Worthington; Freehold NJ) and concentrated in suspension.⁸ A density of 60 x 10⁶ cells/cm³ was used to seed 40 PGA-PLGA constructs with chondrocytes. The cell-polymer constructs were incubated for 4 hours in vitro (37°, 5% CO₂) in order to allow the chondrocytes to adhere to the matrix.

### In Vivo Implantation

All procedures were done in accordance to the Children's Hospital Animal Care Committee protocols. Twenty cell-seeded constructs were implanted in the subcutaneous space of 8-week old nude mice (Jackson laboratories; Bar Harbor, ME) immediately after seeding. The mice were sacrificed 4 and 10 weeks after implantation for analyses.

### In vitro bioreactor system

Twenty cell-polymer constructs were suspended in HAMM's F12 medium (Gibco; New York, NY) containing 10% fetal bovine serum with L-glutamine (292 µg/ml), penicillin (100 µg/ml) and ascorbic acid (50 µg/ml). Five days after seeding, the cell-polymer constructs, which were held in an incubator (37°, 5% CO₂) in static conditions, were suspended freely in a bioreactor system. Five constructs were placed in each magnetically stirred spinner flask (Belico Glass, Vineland, NJ). The medium (150ml), was replaced every other day. Gas was exchanged through loosened side arm caps.⁹

### Analytical Assays

Four and 10 weeks after cell seeding the cell-matrix constructs were retrieved from both, the subcutaneous space of the nude mice and from the bioreactors. The cell-matrix constructs were evaluated for gross wet weight. Samples were fixed for histological analysis with 10% buffered formalin (Fisher Scientific), embedded in paraffin and sectioned. Sections were stained with hematoxylin and eosin, safranin O for glycosaminoglycans (GAG), alcian blue for chondroitin sulfate and Masson's trichrome for cross-linked collagen.

To evaluate collagen content, the Sircol Collagen Assay (Bicolor; Belfast, N. Ireland) was used. In brief, 10mg of tissue (3 stents from each time point; 2 samples from each stent) was suspended in 10mg of pepsin and 0.5 ml of 0.5 molar acetic acid in a shaker at 4°c for 24th. Each pepsin-tissue suspension underwent serial dilutions in acetic acid. Serial dilutions of collagen standard in acetic acid served as a reference curve for collagen concentration. All standards and pepsin-tissue dilutions were mixed with dye and rotated for 30 minutes at room temperature. The pellets were washed with 0.5ml ethanol and 1 ml of alkali reagent was added. Readings were performed using a spectrophotometer (Milton Roy; Rochester, NY) at 540 nm. Acetic acid and alkali reagents only served as controls.

Scanning electron microscopy was performed in order to determine the distribution of the chondrocytes throughout the polymer scaffolds before implantation and after implantation at the 4 and 10-week time points. Specimens were fixed in 1% (v/v) buffered glutaraldehyde and 0.1 % (v/v) buffered formaldehyde for 30 minutes and 24 hours, respectively, dehydrated with a graded ethanol series, and air dried. The dried samples were mounted on aluminum supports and sputter coated with gold. A scanning electron microscope (JOEL, model JSM-35, Peabody, MA) was operated to image specimens with a voltage of 25-kV.

The compressive mechanical properties of the tubular cartilage (n=3) were tested 4 and 10 weeks after cell seeding, using a mechanical tester (model 5542, Instron corp., Canton, MA). A 500-Newton-maximum load cell was used. The longitudinal axis of specimens was compressed until it reached 80% of the initial thickness at a cross-head speed of 0.5 inch/min, and relaxed to its original thickness at the same speed. The compressive modulus was obtained from the slope of the initial linear section of the stress-strain curve.

### RESULTS

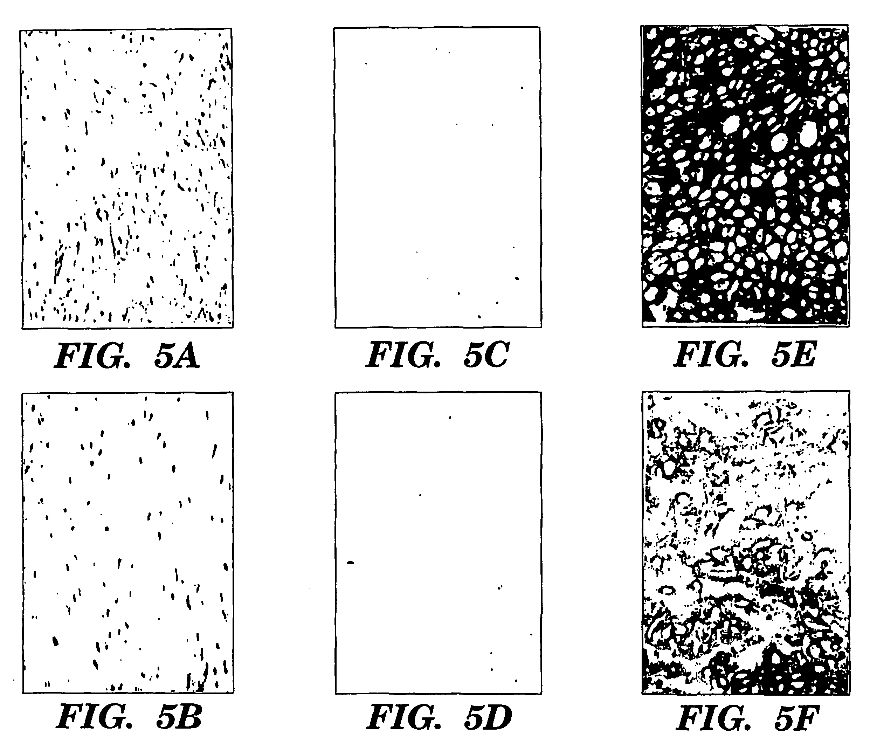

Scanning electron microscopy, which was performed 3 days after cell seeding on the PGA matrices, showed an even distribution of chondrocytes throughout the scaffolds (Figure 2B). The cartilage stents, which were retrieved from both, the subcutaneous space of the athymic mice and from the bioreactors, 4 and 10 weeks after cell seeding, showed grossly a solid, glistening appearance of cartilaginous tissue without ingrowth of cartilage into the luminal region (Figure 3B). Four weeks after cell seeding the constructs engineered in vitro and in vivo had a mean wet weight of 0.42 grams and 0.71 grams, respectively. Ten weeks after cell seeding the constructs engineered in vitro and in vivo had a mean wet weight of 0.53 grams and 0.97 grams, respectively. An increase in mean wet weight was noted from the 4 week to the 10 week time points, 26% for the in vitro constructs and 37% for the in vivo constructs. Histological analyses of all constructs with hematoxylin & eosin at 4 weeks demonstrated an even distribution of chondrocytes on the PGA fibers. An increase in the deposition of extracellular matrix could be seen at 10 weeks without any residual PGA fibers. These findings were confirmed with scanning electron microscopy. Cross-linked collagen content, as demonstrated by Masson's trichrome, was higher in the in vivo explants than in the bioreactor specimens at both time points (Figure 5A and 5B). Safranin O and alcian blue confirmed the deposition of GAG and chondroitin sulfate in all experimental groups. Increased deposition of collagen and GAG was observed in the in vivo groups at both time points (Figure 5C-5F).

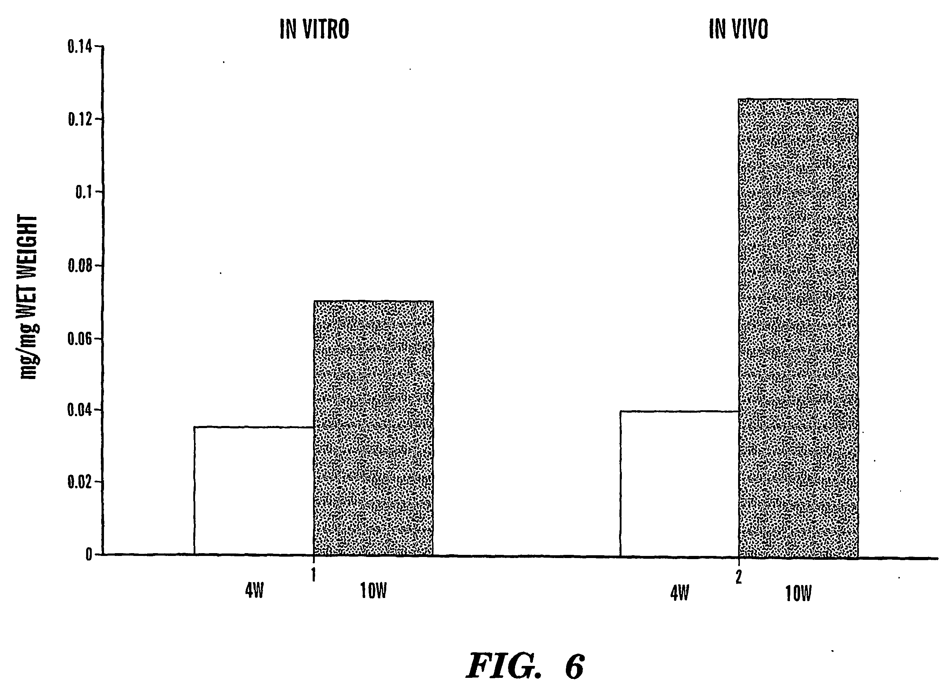

Collagen content, determined by the Sircol biochemical assay, demonstrated that the constructs grown in vitro had an average collagen composition fraction of 3.6% (s.d.±0.22%) of the total wet weight at 4 weeks and of 7% (s.d.±0.41 %) at 10 weeks (Figure 6). The constructs grown in vivo had an average collagen composition fraction of 4% (s.d.±0.33%) at 4 weeks and 12.4% (s.d.±0.9 1%) at 10 weeks (Figure 6).

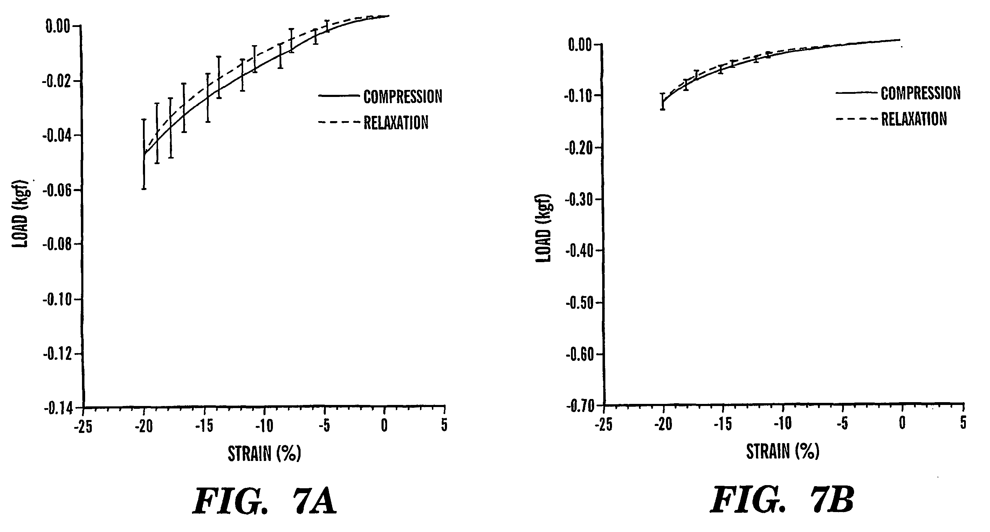

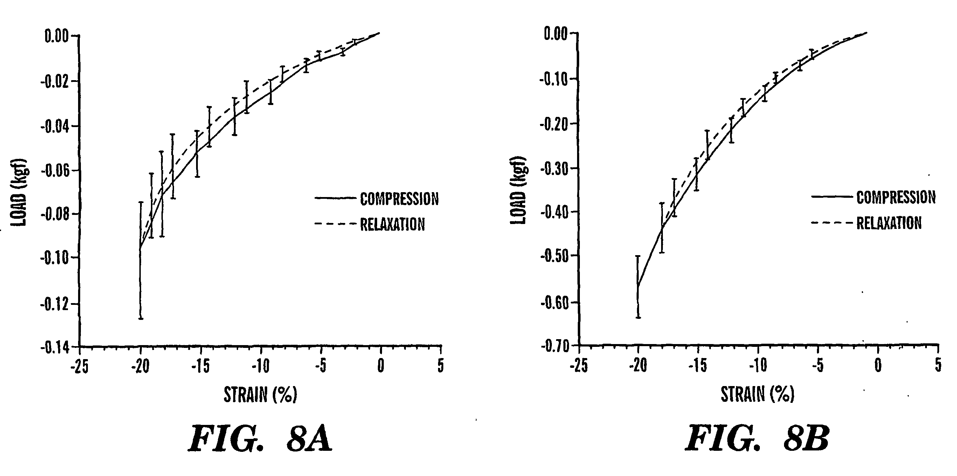

Mechanical testing demonstrated that the cartilaginous cylinders in both groups were readily elastic and withstood high degrees of pressures. The cartilage stents, which were compressed for up to 80% of their initial thickness, returned to their initial state when the load was released. The compression forces required to reduce the diameter of the engineered constructs by 20% were higher in the in vivo specimens than in the in vitro specimens at all time points (4 weeks: 0.101±0.27kgf vs. 0.050±0.013kgf; 10 weeks: 0.570±0.070kgf vs. 0.115±0.017kgf; Figure 7 and 8). The cartilage cylinders engineered in vivo exhibited a 2-fold higher stiffness compared to those engineered in vitro at the 4 week and 10 week time points, respectively (0.007±0.03 kgf versus 0.014±0.003 kgf; 0.015±0.001 kgf versus 0.07±0.008 kgf).

### REFERENCES

1. Atala, A., Cima, L. G., Kim, W. et. al.: Injectable alginate seeded with chondrocytes as a potential treatment for vesicoureteral reflux. J. Urol. **150**:745, 1993.
2. Atala, A., Kim, W., Paige, K. T. et. al.: Endoscopic treatment of vesicoureteral reflux with a chondrocyte-alginate suspension. J. Urol. **152**:641, 1994.
3. Kershen, R. T. and Atala, A.: New advances in injectable therapies for the treatment of incontinence and vesicoureteral reflux. Urol. Clin. North. Am. **26**:811, 1999.
4. Diamond, D. A. and Caldamone, A. A.: Endoscopic correction of vesicoureteral reflux in children using autologous chondrocytes: preliminary results. J. Urol. **162**:1185, 1999.
5. Bent, A. E., Tutrone, R. T., Lloyd, L. K. et. al.: Treatment of intrinsic sphincter deficiency using autologous ear cartilage as a periurethral bulking agent. Paper presented at the 1999 International Continence Society Meeting, Denver, Colorado, 8/22-26/1999.
6. Yoo, J. J., Lee, I. and Atala, A.: Cartilage rods as a potential material for penile reconstruction. J. Urol.**160**:1164, 1998.
7. Yoo, J. J., Park, H. J., Lee, I. Et. al.: Autologous engineered cartilage rods for penile reconstruction. J. Urol. **162**:1119, 1999.
8. Klagsbrun, M.: Large-scale preparation of chondrocytes. Meth. Enzym. **58**:549, 1979.
9. Obradovic, B., Carrier, R. L., Vunjak-Novakovic, G. et. al.: Gas exchange is essential for bioreactor cultivation of tissue engineered cartilage. Biotechnol. Bioeng. **63**:197, 1999.
10. Heyns, C. F., Steenkamp, J. W., de Kock, M.L.S. et. al.: Treatment of male urethral strictures: is repeated dilation or internal urethrotomy useful? J. Urol. **160**:356,1988.
11. Pitkamaki, K. K., Tammela, T. L. J. and Kontturi, M.J.: Recurrence of urethral stricture and late results after optical urethrotomy: comparison of strictures caused by toxic latex catheters and other causes. Scand. J. Urol. Nephrol. **26**: 327, 1992.
12. Steenkamp, J. W., Heyns, C. F. and de Kock, M.L.S.: Internal urethrotomy versus dilation as treatment for male urethral strictures: a prospective, randomized comparison. J. Urol. **157**: 98, 1997.
13. Ravery, V., de la Taille, A., Hoffmann, P. et. al.: Balloon catheter dilatation in the treatment of ureteral and ureteroenteric stricture. J. Endourol. **12**:335, 1998.
14. Ahmed, M., Bishop, M. C., Bates, C. P. et. al.: Metal mesh stents for ureteral obstruction caused by hormone-resistant carcinoma of prostate. J. Endourol. **13**:221, 1999.
15. Wolf, J. S. Jr, Elashry. O. M. and Clayman, R. V.: Long-term results of endoureterotomy for benign ureteral and ureteroenteric strictures. J. Urol. **158**:759, 1997.
16. Milroy, E.J.G., Chapple, C. R., Cooper, J. E. et. al.: A new treatment for urethral strictures. Lancet **1**: 1424, 1988.
17. Yachia, D. and Beyar, M.: Temporarily implanted urethral coil stent for the treatment of recurrent urethral strictures: a preliminary report. J. Urol. **146**: 1001, 1991.
18. Nissenkorn, I.: A simple nonmetal stent for treatment for treatment of urethral strictures: a preliminary report, J. Urol. **154**:1117, 1995.
19. Yachia, D.: Temporary metal stents in bladder outflow obstruction. J. Endourol. **11**:459, 1997.
20. Talja, M., Valimaa, T., Tammela, T. et. al.: Bloabsorbable and biodegradable stents in urology. J. Endourol. **11**:391,1997.
21. Kemppainen, E., Talja, M., Riihela, M. et. al.: A bioresorbable urethral stent: an experimental study. Urol. Res. **21**:235, 1993.
22. Isolato, T., Tammela, T. L. J., Talja, M. et. al.: A bioabsorbable self-expandable, self-reinforced poly-1-lactic acid urethral stent for recurrent urethral strictures: a preliminary report. J. Urol. **160**: 2033, 1998.
23. Stockwell, R. A.: The cell density of human articular and costal cartilage. J. Anat.**101**:753, 1967.
24. Vunjak-Novakovic, G., Martin, I., Obradovic, B. et. Al.: Bioreactor cultivation conditions modulate the composition and mechanical properties of tissue-engineered cartilage. J. Orthop. Res. **17**:130, 1999.

## Claims

1. A method for producing a tissue engineered stent comprising the steps of:
(a) providing a substrate shaped to form a substantially cylindrical construct, the substrate comprising a biodegradable polymer;
(b) contacting said substrate with dissociated chondrocytes capable of adhering thereto and forming cartilage, thereby forming a cell-seeded construct;
(c) maintaining said cell-seeded construct for a growth period in a fluid media suitable for growth of said chondrocytes to form a tissue-engineered stent.

2. The method of claim 1, wherein said chondrocytes are autologous, allogenic or xenogenic.

3. The method of claim 1, wherein said chondrocytes are autologous.

4. A tissue engineered stent comprising a substantially cylindrical construct having a first end and second end; a walled surface disposed between the first end and the second end, wherein the walled surface comprises a biodegradable polymer scaffold seeded with disassociated chondrocytes.

5. The tissue engineered stent of claim 4, wherein the biodegradable polymer scaffold is cultured *in vitro* prior to implantation into a recipient.

6. The tissue engineered stent of either one of claims 4 and 5 wherein the biodegradable polymer scaffold comprises polyglycolic acid.

7. The tissue engineered stent of any one of claims 4, 5 and 6 wherein the scaffold further comprises poly(lactide-co-glycolide) (PLGA).

8. The tissue engineered stent of claim 4, wherein the chondrocytes have been expanded *in vitro.*

9. The tissue engineered stent of claim 4, wherein the stent is for implantation into the urogenital tract.

10. The tissue engineered stent of claim 4, wherein the stent is for implantation into a blood vessel.

11. The method of claim 4, wherein the dissociated chondrocytes are present in an amount sufficient to form cartilage.

12. The method of claim 1, wherein the biodegradable polymer is polyglycolic acid, poly(lactide-co-glycolide) (PLGA), or a copolymer thereof.

## Patentansprüche

1. Ein Verfahren zum Herstellen eines mittels Tissue-Engineering gebildeten Stents, das die folgenden Schritte beinhaltet:
(a) Bereitstellen eines Substrats, das gestaltet ist, um eine im Wesentlichen zylindrische Konstruktion zu bilden, wobei das Substrat ein biologisch abbaubares Polymer beinhaltet;
(b) In-Kontakt-Bringen des Substrats mit dissoziierten Chondrozyten, die in der Lage sind, daran anzuhaften und Knorpel zu bilden, wodurch eine mit Zellen besäte Konstruktion gebildet wird;
(c) Halten der mit Zellen besäten Konstruktion für eine Wachstumsperiode in einem Flüssigkeitsmedium, das für das Wachstum der Chondrozyten geeignet ist, um einen mittels Tissue-Engineering gebildeten Stent zu bilden.

2. Verfahren gemäß Anspruch 1, wobei die Chondrozyten autolog, allogen oder xenogen sind.

3. Verfahren gemäß Anspruch 1, wobei die Chondrozyten autolog sind.

4. Ein mittels Tissue-Engineering gebildeter Stent, der eine im Wesentlichen zylindrische Konstruktion mit einem ersten Ende und einem zweiten Ende beinhaltet, wobei eine mauerartige Oberfläche zwischen dem ersten und dem zweiten Ende angeordnet ist, wobei die mauerartige Oberfläche ein biologisch abbaubares Polymergerüst beinhaltet, das mit dissoziierten Chondrozyten besät ist.

5. Mittels Tissue-Engineering gebildeter Stent gemäß Anspruch 4, wobei das biologisch abbaubare Polymergerüst vor Implantation in einen Empfänger *in vitro* gezüchtet wird.

6. Mittels Tissue-Engineering gebildeter Stent gemäß einem der Ansprüche 4 und 5, wobei das biologisch abbaubare Polymergerüst Polyglykolsäure beinhaltet.

7. Mittels Tissue-Engineering gebildeter Stent gemäß einem der Ansprüche 4, 5 und 6, wobei das Gerüst weiter Poly(lactid-coglycolid) (PLGA) beinhaltet.

8. Mittels Tissue-Engineering gebildeter Stent gemäß Anspruch 4, wobei die Chondrozyten *in vitro* expandiert wurden.

9. Mittels Tissue-Engineering gebildeter Stent gemäß Anspruch 4, wobei der Stent zur Implantation in den Urogenitaltrakt Ist.

10. Mittels Tissue-Engineering gebildeter Stent gemäß Anspruch 4, wobei der Stent zur Implantation in ein Blutgefäß ist.

11. Verfahren gemäß Anspruch 4, wobei die dissoziierten Chondrozyten in einer Menge vorhanden sind, die ausreichend für das Bilden von Knorpel ist.

12. Verfahren gemäß Anspruch 1, wobei das biologisch abbaubare Polymer Polyglykolsäure, Poly(lactid-co-glycolid) (PLGA) oder ein Copolymer davon ist.

## Revendications

1. Une méthode pour produire un stent d'ingénierie tissulaire, comportant les étapes de :
(a) fournir un substrat configuré pour former une structure substantiellement cylindrique, le substrat comportant un polymère biodégradable ;
(b) mettre en contact ledit substrat avec des chondrocytes dissociés capables d'y adhérer et de former du cartilage, formant de ce fait une structure ensemencée de cellules ;
(c) conserver ladite structure ensemencée de cellules pendant une période de croissance dans un milieu fluide convenant à la croissance desdits chondrocytes pour former un stent d'ingénierie tissulaire.

2. La méthode de la revendication 1, dans laquelle lesdits chondrocytes sont autologues, allogéniques ou xénogéniques.

3. La méthode de la revendication 1, dans laquelle lesdits chondrocytes sont autologues.

4. Un stent d'ingénierie tissulaire comportant une structure substantiellement cylindrique ayant une première extrémité et une deuxième extrémité ; une surface en forme de paroi disposée entre la première extrémité et la deuxième extrémité, dans lequel la surface en forme de paroi comporte un échafaudage en polymère biodégradable ensemencé de chondrocytes dissociés.

5. Le stent d'ingénierie tissulaire de la revendication 4, dans lequel l'échafaudage en polymère biodégradable est cultivé in vitro avant implantation dans un receveur.

6. Le stent d'ingénierie tissulaire de l'une ou l'autre des revendications 4 et 5 dans lequel l'échafaudage en polymère biodégradable comporte de l'acide polyglycolique.

7. Le stent d'ingénierie tissulaire de n'importe laquelle des revendications 4, 5 et 6 dans lequel l'échafaudage comporte de plus du poly(lactide-co-glycolide) (PLGA).

8. Le stent d'ingénierie tissulaire de la revendication 4, dans lequel les chondrocytes ont été dilatés in vitro.

9. Le stent d'ingénierie tissulaire de la revendication 4, dans lequel le stent est destiné à l'implantation dans le tractus uro-génital.

10. Le stent d'ingénierie tissulaire de la revendication 4, dans lequel le stent est destiné à l'implantation dans un vaisseau sanguin.

11. La méthode de la revendication 4, dans laquelle les chondrocytes dissociés sont présents dans une quantité suffisante pour former du cartilage.

12. La méthode de la revendication 1, dans laquelle le polymère biodégradable est de l'acide polyglycolique, du poly(lactide-coglycolide) (PLGA), ou un copolymère de ceux-ci.
